# EUROPÄISCHE PATENTSCHRIFT

(11) **EP 0 922 491 B1**
(45) Veröffentlichungstag und Bekanntmachung des Hinweises auf die Patenterteilung: **28.12.2005**
(21) Anmeldenummer: 98122922.2
(22) Anmeldetag: 02.12.1998
(51) Int. Cl.: B01J 23/66, B01J 37/34, C07C 67/055

(54) **Katalysator auf der Basis von Pd, Au und Alkalimetall(e), seine Herstellung und seine Verwendung in der Herstellung von Vinylacetat**
Catalyst based on Pd, Au und alkali metal(s), its preparation and its use in the preparation of vinyl acetate
Catalyseur à base de Pd, Au et d'alcalin (s), sa préparation et son utilisation dans la préparation d'acétate de vinyle

(30) Priorität: 11.12.1997 DE 19754992
(43) Veröffentlichungstag der Anmeldung: 16.06.1999
(73) Patentinhaber: Celanese Chemicals Europe GmbH, 60439 Frankfurt am Main (DE)
(72) Erfinder: Herzog, Bernhard Dr., 46145 Oberhausen (DE)

(56) Entgegenhaltungen:
- DE-A- 19 613 791
- FR-A- 2 677 898
- US-A- 5 332 710

## Beschreibung

Die vorliegende Erfindung betrifft einen Katalysator, der Palladium und/oder dessen Verbindungen, Gold und/oder dessen Verbindungen sowie mindestens eine Alkalimetallverbindung enthält, ein Verfahren zu seiner Herstellung und seine Verwendung zur Herstellung von Vinylacetat in der Gasphase aus Essigsäure, Ethylen und Sauerstoff oder Sauerstoff enthaltenden Gasen.

Aus dem Stand der Technik ist bekannt, daß Vinylacetat in der Gasphase aus Ethylen, Sauerstoff und Essigsäure in Gegenwart von Katalysatoren hergestellt werden kann, die auf einem porösen Trägermaterial (wie etwa Siliziumdioxid) Palladium, Gold sowie Alkalimetallverbindungen enthalten.

Von besonderer Bedeutung für die Aktivität und Selektivität dieser Katalysatoren ist dabei die Verteilung der Edelmetalle auf dem Trägermaterial. Da die Reaktanden der zu katalysierenden Reaktion nicht ohne weiteres in der Lage sind, in die mittleren oder inneren Bereiche des porösen Trägermaterials zu diffundieren, findet die Reaktion im wesentlichen nur an den äußersten oder Oberflächenbereichen des Katalysators statt. Somit tragen die im Inneren oder den Mittelbereichen des Trägers befindlichen Metallkomponenten nicht signifikant zum Reaktionsmechanismus bei, was zu einer Verringerung der Leistung des Katalysators bezogen auf das Gewicht der Edelmetalle führt.

Im Rahmen der Entwicklung leistungsfähiger Katalysatoren für die Vinylacetatproduktion sind daher die Bestrebungen darauf ausgerichtet, Katalysatoren bereitzustellen, bei denen die katalytisch aktiven Edelmetalle in einer Schale auf den Trägerteilchen vorliegen, während der Kern der Trägerteilchen weitgehend frei von Edelmetallen ist. Derartige Schalenkatalysatoren lassen sich prinzipiell durch Imprägnieren des Trägermaterials mit löslichen Edelmetallverbindungen, nachfolgendes Ausfällen unlöslicher Edelmetallverbindungen auf dem Träger durch Einwirkung alkalischer Verbindungen sowie abschließende Reduktion zu den Edelmetallen herstellen.

US-A-4,048,096 beschreibt ein Verfahren zur Herstellung eines Palladium, Gold und Kalium enthaltenden Katalysators für die Vinylacetatproduktion. Zunächst wird der Katalysatorträger mit einer Lösung imprägniert, welche eine Mischung der gelösten Palladium- und Goldsalze enthält. Erfindungswesentlich ist hierbei, daß diese Lösung das gleiche Volumen wie die Poren des Trägermaterials im trockenen Zustand besitzt. Während dieses Imprägnierungsschrittes werden die Trägerteilchen in einem rotierenden Gefäß bewegt. Ohne vorhergehende Trocknung des imprägnierten Trägers werden die Edelmetallsalze auf den Trägerteilchen anschließend durch den Zusatz von Alkalien in unlösliche Verbindungen überführt und somit auf den Trägerteilchen fixiert. Durch eine abschließende Behandlung mit einem Reduktionsmittel werden die Palladium- und Goldverbindungen zu den entsprechenden Metallen reduziert. Nach Aufbringung einer Alkalimetallverbindung in einem weiteren Imprägnierungsschritt wird ein Katalysator erhalten, der die gewünschte Schalenstruktur aus Palladium und Gold mit einer Dicke von 0,5 mm auf der Oberfläche des Trägermaterials aufweist.

Auch die US-A-3,775,342 beschreibt die Herstellung eines Palladium, Gold und Kalium enthaltenden Katalysators zur Vinylacetatproduktion. In diesem Verfahren wird das Trägermaterial in beliebiger Reihenfolge mit zwei Lösungen behandelt, von denen die eine die gelösten Palladium- und Goldsalze enthält und die andere eine alkalisch reagierende Substanz. Nach der Behandlung mit der ersten Lösung wird der Träger in einem Zwischenschritt getrocknet, bevor er mit der zweiten Lösung in Kontakt gebracht wird. Das Volumen beider Lösungen entspricht jeweils dem Porenvolumen des Trägermaterials.

DE-A-196 13 791 behandelt die Herstellung von Vinylacetatkatalysatoren, die neben Palladium und Gold noch zusätzlich Bor oder Borverbindungen enthalten.

US-A-5,332,710 offenbart ferner die Herstellung eines Katalysators zur Produktion von Vinylacetat, wobei man die unlöslichen Edelmetallsalze ebenfalls durch Alkalienzusatz auf den Trägerteilchen niederschlägt. Hierzu werden die Trägerteilchen in der alkalisch reagierenden Lösung untergetaucht und ab Beginn der Niederschlagung mindestens eine halbe Stunde in einer Trommel rotierend bewegt. Dieses Verfahren wird als "rotation-immersion" bezeichnet.

Die Anwendung der Mikrowellenbestrahlung bei der Katalysatorherstellung ist aus FR-A-2 677 898 bekannt. Danach wird die nach Imprägnierung des Trägermaterials, Calcinierung und Reduktion erhaltene Vorstufe bei erhöhter Temperatur von 200°C in Gegenwart von Wasserstoff einer Mikrowellenbestrahlung unterzogen. Die nach FR-A-2 677 898 bekannten Katalysatoren eignen sich zur Konvertierung von Kohlenwasserstoffen in Gegenwart von Wasserstoff.

Die gemäß den zuvor genannten Verfahren hergestellten Katalysatoren führen bei der Herstellung von Vinylacetat häufig noch zu einer unerwünscht hohen Bildung von Abbau- und Nebenprodukten, wie z.B. Kohlendioxid, wodurch Aktivität und Selektivität der Gesamtreaktion negativ beeinflußt werden.

Vor dem Hintergrund, daß es sich bei Vinylacetat um ein im großtechnischen Maßstab hergestelltes Massenprodukt handelt, besteht somit die Aufgabe der voriiegenden Erfindung darin, einen Katalysator zur Verfügung zu stellen, der bei der Herstellung von Vinylacetat in der Gasphase eine noch weiter verbesserte Selektivität aufweist.

Gegenstand der Erfindung ist ein Verfahren zur Herstellung eines Katalysators zur Produktion von Vinylacetat in der Gasphase aus Ethylen, Essigsäure und Sauerstoff oder Sauerstoff enthaltenden Gasen, der Palladium und/oder dessen Verbindungen, Gold und/oder dessen Verbindungen sowie Alkalimetallverbindungen auf einem teilchenförmigen, porösen Träger enthält, wobei der Katalysator hergestellt wird, indem man
a) den Träger mit löslichen Palladium- und Goldverbindungen imprägniert,
b) die löslichen Palladium- und Goldverbindungen durch Zugabe einer alkalisch reagierenden Lösung auf dem Träger in unlösliche Palladium- und Goldverbindungen überführt,
c) die unlöslichen Palladium- und Goldverbindungen auf dem Träger durch ein Reduktionsmittel in flüssiger oder gasförmiger Phase reduziert,
d) den Träger mit mindestens einer löslichen Alkalimetallverbindung imprägniert und
e) den Träger abschließend bei höchstens 150°C trocknet,

Dieses Verfahren ist dadurch gekennzeichnet, daß der Katalysator vor, während oder nach einem der Verfahrensschritte a) bis e) mit Mikrowellen bestrahlt wird.

Gegenstand der Erfindung ist ferner ein Katalysator zur Herstellung von Vinylacetat in der Gasphase aus Ethylen, Essigsäure und Sauerstoff oder Sauerstoff enthaltenden Gasen, der Palladium und/oder dessen Verbindungen, Gold und/oder dessen Verbindungen sowie Alkalimetallverbindungen auf einem teilchenförmigen, porösen Träger enthält, erhältlich, indem man
a) den Träger mit löslichen Palladium- und Goldverbindungen imprägniert,
b) die löslichen Palladium- und Goldverbindungen durch Zugabe einer alkalisch reagierenden Lösung auf dem Träger in unlösliche Palladium- und Goldverbindungen überführt,
c) die unlöslichen Palladium- und Goldverbindungen auf dem Träger durch ein Reduktionsmittel in flüssiger oder gasförmiger Phase reduziert,
d) den Träger mit mindestens einer löslichen Alkalimetallverbindung imprägniert und
e) den Träger abschließend bei höchstens 150°C trocknet,
dadurch gekennzeichnet, dass der Katalysator nach dem Verfahrensschritt e) mit Mikrowellen bestrahlt wird.

Ein weiterer Gegenstand der Erfindung betrifft ein Verfahren zur Herstellung von Vinylacetat in der Gasphase aus Ethylen, Essigsäure und Sauerstoff und/oder Sauerstoff enthaltenden Gasen in Gegenwart eines Katalysators, erhältlich, indem man
a) den Träger mit löslichen Palladium- und Goldverbindungen imprägniert,
b) die löslichen Palladium- und Goldverbindungen durch Zugabe einer alkalisch reagierenden Lösung auf dem Träger in unlösliche Palladium- und Goldverbindungen überführt,
c) die unlöslichen Palladium- und Goldverbindungen auf dem Träger durch ein Reduktionsmittel in flüssiger oder gasförmiger Phase reduziert,
d) den Träger mit mindestens einer löslichen Alkalimetallverbindung imprägniert und
e) den Träger abschließend bei höchstens 150°C trocknet,
dadurch gekennzeichnet, dass der Katalysator nach dem Verfahrensschritt e) mit Mikrowellen bestrahlt wird.

Überraschenderweise führen die erfindungsgemäßen Katalysatoren bei der Herstellung von Vinylacetat nicht nur zu einer verbesserten Selektivität der Reaktion, sondern ermöglichen auch eine höhere Raum-Zeit-Ausbeute.

Die Trägerteilchen des erfindungsgemäßen Katalysators können eine beliebige geometrische Form haben, beispielsweise die Form von Kugeln, Tabletten, Zylindern, Ringen oder Sternen in regelmäßiger oder unregelmäßiger Ausführung. Die Abmessungen der Trägerteilchen, d.h. der Durchmesser bzw. die Länge und Dicke liegen im allgemeinen zwischen 1 und 10 mm, insbesondere bei 3 bis 9 mm. Bevorzugt werden Trägerteilchen in Kugelform mit einem Durchmesser von 4 bis 8 mm verwendet.

Als Träger können die bekannten inerten Trägermaterialien wie Kieselsäure, Aluminiumoxid, Alumosilikate, Silikate, Titanoxid, Zirkonoxid, Titanate, Siliciumcarbid und Kohle eingesetzt werden. Geeignet sind ferner die durch Flammenhydrolyse von Siliciumtetrachlorid erhaltenen sogenannten pyrogenen Kieselsäuren oder die durch Flammenhydrolyse von Siliciumtetrachlorid und einem anderen Metallchlorid wie z.B. Aluminiumchlorid erhaltenen pyrogenen SiO₂-MₓO_{y}-Gemische (US-A-3,939,199 und EP-A-0 723 810). Bevorzugt werden Kieselsäure (SiO₂), Baddelyt (ZrO₂) sowie SiO₂-Al₂O₃-Gemische als Trägermaterial eingesetzt. Im Falle der pyrogenen Trägermaterialien eignen sich besonders die in der DE-OS-38 03 895 sowie DE-OS-39 12 504 beschriebenen Preßlinge.

Entscheidend für die Eignung als Trägermaterial ist es, daß.das Trägermaterial unter den Reaktionsbedingungen des katalytischen Prozesses der Vinylacetatherstellung, insbesondere unter dem Einfluß der Essigsäure, seine mechanische Festigkeit behält.
Besonders geeignet sind Träger der vorgenannten Art mit einer spezifischen Oberfläche von 50 bis 400 m²/g (gemessen nach der BET-Methode) und einem mittleren Porenradius von 50 bis 2000 Å (gemessen mittels der Quecksilber-Porosimetrie).

In Schritt a) des erfindungsgemäßen Verfahrens, dem sogenannten Imprägnierungsschritt, werden die Trägerteilchen mit den löslichen Palladium- und Goldverbindungen imprägniert. Als Palladium- und Goldverbindungen kommen alle Salze und Komplexe in Betracht, die in den nachfolgend beschriebenen Lösungsmitteln löslich sind, ferner einer Fällung als Hydroxid oder Oxid zugänglich sind und im fertigen Katalysator -gegebenenfalls nach einem Waschschritt- keine die Leistungsfähigkeit des Katalysators beeinträchtigenden Stoffe hinterlassen.

Als Palladiumverbindungen sind beispielsweise Palladium-(II)-chlorid, Natrium- oder Kalium-Palladium-(II)-chlorid, Palladium-(II)-nitrat, -nitrit, -sulfat, -oxidhydrat, -oxalat, -acetylacetonat oder acetoacetat geeignet. Ferner können auch Palladiumsalze der aliphatischen Monocarbonsäuren mit 2 bis 5 Kohlenstoffatomen, bevorzugt das Palladium-(II)-acetat, eingesetzt werden. Gold-(III)-chlorid, Gold-(III)-acetat, Tetrachlorogold-(III)-säure sowie deren Alkalimetallsalze können als lösliche Goldverbindungen verwendet werden. Im allgemeinen werden von diesen Verbindungen solche Mengen eingesetzt, daß der fertige Katalysator 2 bis 14 g/l, bevorzugt 4 bis 8 g/l, Palladium sowie 1 bis 8 g/l, bevorzugt 2 bis 5 g/l, Gold enthält.

Als Lösungsmittel für die Palladium- und Goldverbindungen wie auch für die in Schritt d) aufzubringenden Alkalimetallverbindungen sind alle Verbindungen geeignet, in denen die gewählten Salze löslich sind und die nach der Imprägnierung in einem optionalen Trocknungsschritt leicht wieder zu entfernen sind. Geeignet sind vor allem Wasser oder unsubstituierte Carbonsäuren mit 2 bis 10 Kohlenstoffatomen, wie Essigsäure, Propionsäure, n- und iso-Buttersäure sowie die n- und iso-Valeriansäuren. Wegen ihrer günstigen physikalischen Eigenschaften und auch aus wirtschaftlichen Gründen wird als Carbonsäure vorzugsweise Essigsäure eingesetzt. Die Verwendung eines zusätzlichen Lösungsmittels ist dann zweckmäßig, wenn eine Carbonsäure eingesetzt wird, in der die Palladium- und Goldverbindungen nicht ausreichend löslich sind. So läßt sich z.B. Palladium-(II)-chlorid in einer wäßrigen Essigsäure wesentlich besser lösen als in Eisessig. Als zusätzliche Lösungsmittel kommen diejenigen in Betracht, die inert und gleichzeitig mit der Carbonsäure mischbar sind, z.B. Wasser, Ether, wie z.B. Tetrahydrofuran oder Dioxan, oder Kohlenwasserstoffe wie z.B. Benzol.

Im Rahmen der Imprägnierung des Trägermaterials können für jedes der aufzubringenden Metalle verschiedene Salze des betreffenden Metalls verwendet werden, vorzugsweise geht man jedoch jeweils nur von einem Salz pro Metall aus.

Die Imprägnierung des Trägermaterials mit den löslichen. Palladium- und Goldverbindungen in Schritt a) kann unter Verwendung einer Lösung erfolgen, die gleichzeitig alle löslichen Palladium- und Goldverbindungen enthält. Hierbei kann das Trägermaterial ein- oder mehrmals mit dieser Lösung getränkt werden. Da die eingesetzte Menge an Palladium- und Goldverbindungen bei ein- und mehrmaliger Tränkung identisch sein sollte, ist bei mehrmaliger Tränkung das Gesamtvolumen der Lösung entsprechend aufzuteilen. Bevorzugt ist die einmalige Tränkung mit dem Gesamtvolumen der Lösung.

In einer alternativen Ausführungsform kann die Imprägnierung des Trägermaterials aber auch unter Verwendung zweier getrennter Lösungen, von denen die eine die Palladiumverbindungen und die andere die Goldverbindungen enthält, durchgeführt werden. In diesem Fall können die beiden Lösungen entweder gleichzeitig oder aber auch in beliebiger Reihenfolge nacheinander mit dem Trägermaterial in Kontakt gebracht werden, wobei im letzteren Fall der Träger nach der Imprägnierung mit der ersten Lösung getrocknet werden muß.

Für eine effektive Imprägnierung soll das Gesamtvolumen der Edelmetallsalzlösung bzw. der beiden Edelmetallsalzlösungen zusammen etwa 90-100%, bevorzugt 95-100% und insbesondere 98-99%, des Porenvolumens des Trägermaterials im trockenen Zustand betragen. In der Praxis kann dabei auch so verfahren werden, daß die Trägerteilchen mit einem Überschuß der Edelmetallsalzlösung überschichtet werden und die überschüssige Lösung anschließend abgegossen oder abfiltriert wird. Bevorzugt wird aber nur die oben angegebene, ungefähr dem Porenvolumen des Katalysatorträgers entsprechende Menge an Lösung zugegeben.

Es hat sich als vorteilhaft erwiesen, die Trägerteilchen während der Imprägnierung zwecks Erzielung einer innigen Durchmischung zu bewegen. Dies kann mittels eines sich drehenden oder bewegten Kolbens oder einer Mischtrommel erreicht werden. Die Drehgeschwindigkeit bzw. allgemein die Intensität der Bewegung sollte dabei einerseits ausreichend sein, um eine vollständige Benetzung der Trägerteilchen mit der Imprägnierlösung zu erzielen, andererseits darf sie aber nicht so groß sein, daß ein merklicher Abrieb des Trägermaterials eintri.tt.

Gegebenenfalls wird das imprägnierte Trägermaterial bei Temperaturen von höchstens 150°C, bevorzugt 80-150°C und insbesondere 100-150°C getrocknet. Diese Trocknung kann beispielsweise in einem Gebläsetrockner im Heißluftstrom erfolgen oder aber in einem Trockenschrank in einem Inertgasstrom, insbesondere in einem Stickstoff- oder Kohlendioxidstrom. Gegebenenfalls wird unter vermindertem Druck, vorzugsweise bei 0,01-0,08 MPa, getrocknet.

In Schritt b), dem sogenannten Fixierschritt, werden die auf den Trägerteilchen befindlichen, löslichen Palladium- und Goldverbindungen unter Einwirkung einer alkalisch reagierenden Lösung in unlösliche Verbindungen überführt und damit auf dem Träger fixiert. Hierbei wird angenommen, daß es sich bei den unlöslichen Verbindungen um die Hydroxide und/oder Oxide der Edelmetalle handelt.

Als alkalische Lösung kommt dabei jede Lösung in Betracht, die in der Lage ist, die löslichen Palladium- und Goldverbindungen in unlösliche Verbindungen zu überführen. Als alkalische Reagenzien können Alkalihydroxide, Alkalisilikate und Alkalicarbonate eingesetzt werden. Bevorzugt ist eine wäßrige Lösung der Alkalimetallhydroxide, insbesondere von Kalium- oder Natriumhydroxid. Auch wäßrige Lösungen, die Borverbindungen enthalten, können als alkalische Lösungen verwendet werden. Hierbei sind besonders wäßrige Lösungen von Natriumtetraborat-Dekahydrat ("Borax"), Kaliumtetraborat oder Mischungen aus Alkalilauge und Borsäure geeignet. Die alkalische Lösung kann Puffereigenschaften aufweisen.

Die Menge der in der wäßrigen Lösung enthaltenen alkalischen Verbindung ist zweckmäßigerweise so zu wählen, daß sie für die stöchiometrische Umsetzung der aufgebrachten löslichen Palladium- und Goldverbindungen mindestens ausreicht. Es kann aber auch ein Überschuß der alkalischen Verbindung eingesetzt werden, wobei dieser Überschuß üblicherweise bei der 1-10-fachen Menge, bezogen auf die stöchiometrisch erforderliche Menge, liegt.

Im folgenden werden zwei geeignete Methoden I und II für die Durchführung des Fixierschrittes b) beschrieben, die bei der Herstellung des erfindungsgemäßen Katalysators Anwendung finden können.

Bei Methode I wird das gemäß Schritt a) imprägnierte Trägermaterial ausreichend lange in eine alkalische Lösung gegeben, deren Konzentration so beschaffen ist, daß die gewünschten, unlöslichen Edelmetallverbindungen ausgefällt werden. Das Volumen der alkalischen Lösung wird so gewählt, daß es zur vollständigen Bedeckung und Untertauchung der imprägnierten Trägerteilchen ausreicht. Ferner werden die in der alkalischen Lösung untergetauchten, imprägnierten Trägerteilchen beginnend mit der Abscheidung der unlöslichen Palladium- und Gold-verbindungen rotierend bewegt, wobei die Rotation mindestens eine halbe Stunde, vorzugsweise eine Stunde und maximal bis zu 4 Stunden dauern sollte. Diese Fixiermethode wird als "rotation - immersion" bezeichnet und ist in der US-A-5,332,710, auf die hiermit direkt Bezug genommen wird, detailliert beschrieben.

In dem Fall, daß die nachfolgend beschriebene Methode II zur Fixierung der Palladium- und Goldverbindungen auf den Trägerteilchen angewandt wird, sollte der in Schritt a) imprägnierte Träger vor dem Fixierschritt b) getrocknet werden.

Der Fixierschritt b) besteht bei Methode II aus mindestens zwei getrennten Stufen der Behandlung mit der alkalischen Fixierlösung. In der ersten Fixierstufe wird der imprägnierte und dann getrocknete Träger mit der alkalischen Fixierlösung in Kontakt gebracht. Das Volumen dieser ersten Fixierlösung entspricht dem Porenvolumen und damit dem Absorptionsvermögen des Trägermaterials im trockenen Zustand. Die Menge der darin enthaltenen alkalischen Verbindungen soll so bemessen sein, daß das molare Verhältnis von Alkalimetall aus der alkalischen Verbindung zu Anionen aus dem löslichen Metallsalz im Bereich von 0,7:1 bis 2:1 liegt. Die alkalische Fixierlösung wird zum Aufsaugen auf die Trägerteilchen gegossen, und diese werden dann bis zu 24 Stunden, vorzugsweise 2-8 Stunden, stehen gelassen.

Die zweite Fixierstufe kann bei dieser Methode II in zwei Varianten A) und B) durchgeführt werden. Bei beiden Varianten beträgt das molare Verhältnis zwischen dem Alkalimetall aus der alkalischen Verbindung zum Anion aus dem Metallsalz in der Fixierlösung etwa 0,2:1 bis 2:1.

Gemäß Variante A) der Methode II werden die ungetrockneten Trägerteilchen einer zweiten Fixierlösung ausgesetzt, wobei das Lösungsvolumen die Träger mindestens gerade abdecken soll. Die alkalische Fixierlösung wird zum Aufsaugen auf die Trägerteilchen gegossen, und diese werden dann bis zu 16 Stunden, mindestens jedoch 2 Stunden und vorzugsweise mindestens 4 Stunden stehen gelassen.

Nach Variante B) werden die Träger nach dem Kontakt mit der ersten Fixierlösung im zweiten Schritt gemäß dem rotation-immersion Verfahren der US-A-5,332,710 behandelt. Hierbei werden die Träger in der alkalischen Fixierlösung der zweiten Stufe untergetaucht und gleichzeitig rotierend bewegt. Die Rotation sollte mindestens eine halbe Stunde, vorzugsweise eine Stunde und maximal bis zu 4 Stunden dauern.

Unabhängig von der Variante A) oder B) kann die Behandlung in der zweiten Fixierstufe zu der Behandlung in der ersten Stufe dahingehend äquivalent sein, daß eine Fixierlösung der gleichen Konzentration verwendet wird und das Volumen der zweiten Fixierlösung ebenfalls dem Porenvolumen und damit dem Absorptionsvermögen des Trägermaterials im trockenen Zustand enspricht. Vorzugsweise liegt das molare Gesamtverhältnis von Alkalimetall zu Anion aus dem Metallsalz für beide Fixierstufen zusammen im Bereich von 1,1:1 bis 3,3:1.

Im Anschluß an den Fixierschritt von Methode I oder den letzten Fixierschritt von Methode II können die Träger mit Wasser gewaschen werden, vorzugsweise mit destilliertem Wasser, um etwaige noch auf dem Trägermaterial befindliche Anionen, wie z.B. Chloride, zu entfernen, die vom Imprägnierschritt herrühren und durch die Abscheidung der Edelmetalle freigesetzt wurden. Ferner wird durch diese Wäsche auch ein gegebenenfalls noch vorhandener Überschuß der alkalischen Verbindung entfernt.

Gegebenenfalls wird das imprägnierte Trägermaterial nach dem Fixierschritt bei Temperaturen von höchstens 150°C, bevorzugt 80-150°C und insbesondere 100-150°C getrocknet. Diese Trocknung kann beispielsweise in einem Gebläsetrockner im Heißluftstrom erfolgen oder aber in einem Trockenschrank in einem Inertgasstrom, insbesondere in einem Stickstoff- oder Kohlendioxidstrom. Gegebenenfalls wird unter vermindertem Druck, vorzugsweise bei 0,01-0,08 MPa, getrocknet. Eine solche Trocknung ist an dieser Stelle besonders dann vorteilhaft, wenn der nachfolgend beschriebene Reduktionsschritt c) in der Gasphase durchgeführt wird. Im Falle der Reduktion in flüssiger Phase ist dagegen eine vorhergehende Trocknung nicht notwendig.

In Schritt c) wird der Träger sowie die darauf niedergeschlagenen unlöslichen Palladium- und Goldverbindungen mit einem Reduktionsmittel behandelt, um die ausgefällten Palladium- und Goldverbindungen in die metallische Form zu überführen. Diese Reduktion kann zum einen in.der flüssigen Phase bei einer Temperatur von 0-90°C, vorzugsweise 15-25°C, durchgeführt werden. Als Reduktionsmittel werden dabei zum Beispiel Hydrazin, Ameisensäure oder ein Alkaliborhydrid, bevorzugt Natriumborhydrid, eingesetzt. Zum anderen ist auch eine Reduktion in der Gasphase mit Wasserstoff, Ethylen, Propylen, Isobutylen, Butylen oder anderen Olefinen als Reduktionsmittel möglich. In diesem Fall ist es vorteilhaft, die Reaktion bei einer erhöhten Temperatur von 40-260°C, vorzugsweise 70-200°C, durchzuführen. Es ist ferner zweckmäßig, das Reduktionsmittel mit einem Inertgas zu verdünnen. Als Inertgas kann beispielsweise Stickstoff, Kohlendioxid oder ein Edelgas verwendet werden. Üblicherweise enthält ein dergestalt mit Inertgas verdünntes Reduktionsmittel 0,01-50 Vol%, vorzugsweise 0,5-20 Vol% Reduktionsmittel. Unabhängig davon, ob die Reduktion in der flüssigen oder der Gasphase durchgeführt wird, sollte das Reduktionsmittel bezogen auf den zu reduzierenden Katalysator im Überschuß zugegeben werden, um sicherzustellen, daß die Gesamtheit der unlöslichen Edelmetallverbindungen in die metallische Form überführt wird.

Im Anschluß an die Reduktion können die Trägerteilchen erneut ein- oder mehrmals gewaschen werden, vorzugsweise mit destilliertem Wasser, um störende Anionen, wie z.B. Chloride, sowie Reste der verwendeten alkalischen Verbindung zu entfernen. Der Waschvorgang kann auch dazu dienen, Reste des Reduktionsmittels aus Schritt c) zu entfernen.

Anschließend kann der Katalysator erneut getrocknet werden, wobei die Trocknungsbedingungen analog zu den Bedingungen einer Trocknung im Anschluß an den Fixierschritt b) gewählt werden sollten.

Schließlich ist der Zusatz mindestens einer Alkalimetallverbindung notwendig. Der Katalysator wird daher in Schritt d) vorzugsweise mit einer wäßrigen Lösung einer Alkalimetallverbindung imprägniert. Als Alkalimetallverbindungen können Natrium-, Kalium-, Rubidium- oder Cäsiumverbindungen eingesetzt werden, bevorzugt sind Kaliumverbindungen. Als Anionen dieser Alkalimetallverbindungen sind vor allem Carboxylate, insbesondere Acetate oder Propionate geeignet. Besonders bevorzugt ist die Verwendung von Kaliumacetat. Einsetzbar sind allerdings auch Verbindungen, die unter den Reaktionsbedingungen Alkalimetallacetate freisetzen, d.h. im Fall der Verwendung von Essigsäure als Lösungsmittel sind dies die Alkalimetallhydroxide, -oxide oder -carbonate. Bei dieser Imprägnierung wird prinzipiell in der gleichen Weise verfahren wie bei der Imprägnierung des Trägermaterials in Schritt a). Für die einsetzbaren Lösungsmittel gelten die gleichen Bedingungen und Definitionen wie im Fall der Lösungen im Imprägnierschritt a). Die Alkalimetallverbindung wird in einer solchen Menge verwendet, daß der Katalysator im Anschluß an den nachfolgend beschriebenen Trocknungsschritt 0,1-10 Gew% Alkalimetall, bevorzugt 1-4 Gew% Alkalimetall, insbesondere Kalium, bezogen auf die Gesamtmasse des Katalysators, enthält.

Abschließend wird der Katalysator in Schritt e) bei Temperaturen von höchstens 150°C, bevorzugt 80-150°C und insbesondere 100-150°C getrocknet. Diese Trocknung kann beispielsweise in einem Gebläsetrockner im Heißluftstrom erfolgen oder aber in einem Trockenschrank in einem Inertgasstrom, insbesondere in einem Stickstoff- oder Kohlendioxidstrom. Gegebenenfalls wird unter vermindertem Druck, vorzugsweise bei 0,01-0,08 MPa, getrocknet.

Das zuvor beschriebene, die Schritte a) bis e) umfassende Verfahren ist dadurch gekennzeichnet, daß der Katalysator vor, während oder nach einem der Schritte a) bis e) mit Mikrowellen bestrahlt wird.

Eine Ausführungsform des erfindungsgemäßen Verfahrens besteht darin, den unbehandelten Katalysatorträger vor dem Imprägnierungsschritt a) der Mikrowellenbestrahlung auszusetzen.

Alternativ dazu kann der Katalysator nach dem Imprägnierungsschritt a) und vor dem Fixierungsschritt b) mit den Mikrowellen bestrahlt werden.

Der Einsatz der Mikrowellen ist auch während des Reduktionschrittes c) möglich, insbesondere dann, wenn die Reduktion in der Gasphase durchgeführt wird.

Ferner kann der Katalysator auch erst im Anschluß an Schritt e) mit Mikrowellen bestrahlt und auf diese Weise getempert werden.

Die Bestrahlung des Katalysators erfolgt üblicherweise bei einer Strahlungsfrequenz von 300 MHz bis 30 GHz, vorzugsweise wird bei 2.45 GHz gearbeitet. Bewährt hat sich hierbei eine Strahlungsleistung von 10-2000 W, bevorzugt 180-900 W, insbesondere 300-600 W, sowie eine Bestrahlungsdauer von bis zu 10 h, bevorzugt 1-60 min, insbesondere 5-15 min.

Der gemäß den Schritten a) bis e) des erfindungsgemäßen Verfahrens sowie der erfindungswesentlichen Mikrowellenbestrahlung herstellbare Katalysator enthält, bezogen auf die Gesamtmasse des Katalysators, 0.2 - 2.5 Gew%, vorzugsweise 0.6 - 1.5 Gew%, Palladium, 0.2 - 2.5 Gew%, vorzugsweise 0.3 - 1.0 Gew%, Gold sowie 0.1 - 10 Gew% Alkalimetall, vorzugsweise 1.0 - 4.0 Gew% Alkalimetall, insbesondere Kalium.

Zur Herstellung von Vinylacetat leitet man Essigsäure, Ethylen und Sauerstoff oder Sauerstoff enthaltende Gase bei Temperaturen von 100 bis 220 °C, vorzugsweise 120 bis 200 °C, und Drücken von 0,1 bis 2,5 MPa, vorzugsweise 0,1 bis 2 MPa, über den erfindungsgemäßen Katalysator. Hierbei können nicht umgesetzte Komponenten im Kreis geführt werden. In manchen Fällen ist auch eine Verdünnung des Reaktionssystems mit inerten Gasen wie Stickstoff oder Kohlendioxid vorteilhaft. Besonders Kohlendioxid eignet sich zur Verdünnung bei einer Kreislauf-Fahrweise, da es bereits während der Reaktion gebildet wird.

Es hat sich bewährt, die Herstellung des Vinylacetats in einem Rührreaktor, einem sogenannten Berty-Reaktor, in Kreislauffahrweise in der Gasphase bei einem konstanten Sauerstoffumsatz von etwa 45% durchzuführen. Zunächst wird der Katalysator in den Reaktor eingefüllt. Anschließend wird eine gemessene Menge an Essigsäure sowie Ethylen und Sauerstoff verdünnt mit Stickstoff hinzugegeben und die Temperatur mit Hilfe eines Heizmantels auf den gewünschten Wert erhöht. Die Reaktion wird üblicherweise nach etwa 18 Stunden beendet, sofern eine Temperatur eingestellt werden konnte, bei der der Sauerstoffumsatz konstant bei 45% liegt. Die Produktgemischzusammensetzung wird mittels Gaschromatographie ermittelt.

Die mit den erfindungsgemäßen Katalysatoren erzielbare höhere Selektivität sowie größere Raum-Zeit-Ausbeute kann in der Praxis in zweierlei Weise genutzt werden: Zum einen dahingehend, daß es in bestehenden Anlagen unter Beibehaltung aller übrigen Reaktionsbedingungen möglich ist, eine größere Menge Vinylacetat pro · Volumeneinheit und Zeiteinheit zu produzieren. Aufgrund der höheren Selektivität weist das aus dem Reaktor entnommene Produktgemisch ferner einen höheren Vinylacetatanteil und weniger Nebenprodukte, insbesondere Kohlendioxid auf. Hierdurch wird die Aufarbeitung, d.h. die Gewinnung des Vinylacetats erleichtert, weil z.B. die Menge des abzutrennenden Kohlendioxids geringer ist und entsprechend auch der mit der Kohlendioxid-Abtrennung verbundene Verlust an mitausgetragenem Ethylen sinkt. Auf diese Weise kann Edukt eingespart werden. Die prinzipielle Aufarbeitung des Produktgemisches im Anschluß an die VinylacetatHerstellung ist beispielsweise in der EP-A-0 423 658 beschrieben.

Die zweite Möglichkeit zur Nutzung der verbesserten Eigenschaften der erfindungsgemäßen Katalysatoren besteht darin, die Reaktionstemperatur der Vinylacetat-Herstellung bei gleichbleibender Raum-Zeit-Ausbeute senken zu können. Eine geringere Reaktionstemperatur wirkt sich wiederum positiv auf die Gesamtstandzeit des Katalysators aus.

### Beispiele 1 und 2:

Als Katalysatorträger wird kugelförmiges Siliziumdioxid der Firma Südchemie AG mit einem Durchmesser von 7 mm und einem Porenvolumen von 324 ml Wasser/g verwendet. 250 ml des Trägers werden mit einer wäßrigen Lösung imprägniert, die Natriumtetrachloropalladat und Natriumtetrachloroaurat enthält. Anschließend werden die Träger bei einer Temperatur, die 100 °C nicht übersteigt, in warmer Luft getrocknet. Die so behandelten Träger werden mit einer wässrigen Natriumhydroxidlösung imprägniert. Das Volumen der Natriumhydroxidlösung ist gleich der Trockenabsorptionsfähigkeit des Trägers. Nach der ersten Stufe wird der mit Base behandelte Träger 4 h lang stehen gelassen und anschließend in eine zweite Natriumhydroxidlösung gegossen. Das Volumen dieser zweiten Natriumhydroxidlösung ist ebenfalls gleich der Trockenabsorptionsfähigkeit des Trägermaterials. Nach der zweiten Behandlung wird das mit Base behandelte Material für eine zusätzliche Dauer von etwa 16 h stehen gelassen. Nach der Fixierung wird das mit Base behandelte Material gründlich mit destilliertem Wasser gewaschen. Der Katalysator wird in einem konstanten Stickstoffstrom bei einer Temperatur von nicht mehr als 150°C getrocknet. Die getrockneten Katalysatoren werden anschließend bei einer Temperatur von 150°C mit Ethylen reduziert. Das reduzierende Gas enthält 5% Ethylen in Stickstoff und wird 5 h lang bei Atmosphärendruck über die Katalysatoren geleitet. Der reduzierte Katalysator wird mit einer wässrigen Lösung, die 10 g Kaliumacetat enthält, bei einem Lösungsvolumen, das dem Absorptionsvermögen des Trägers entsprach, imprägniert. Die Katalysatoren werden bei einer Temperatur von nicht mehr als 150°C getrocknet. Im Anschluß hieran wird der Katalysator bei Raumtemperatur 10 Minuten mit Mikrowellen einer Strahlungsfrequenz von 2.45 GHz und einer Strahlungsleistung von 600 W bestrahlt.

### Vergleichsbeispiel:

Die Herstellung des Katalysators erfolgt wie im Beispiel 1 beschrieben, im Anschluß an die letzte Trocknung unterbleibt jedoch die Bestrahlung mit Mikrowellen.

### Herstellung von Vinylacetat:

Die in den Beispielen 1 und 2 sowie dem Vergleichsbeispiel hergestellten Katalysatoren werden zur Herstellung von Vinylacetat in der Gasphase aus Ethylen, Essigsäure und Sauerstoff enthaltenden Gasen in einem Berty-Reaktor eingesetzt.
Die Versuchsergebnisse sind nachfolgend in Tabelle 1 zusammengefaßt.

**Tabelle 1:**

| Herstellung von Vinylacetat unter Einsatz der in den Beispielen 1 und 2 sowie dem Vergleichsbeispiel erhaltenen Katalysatoren | | |
|---|---|---|
| Beispiel | Raum-Zeit-Ausbeute (g V A M / L _{katalysator} h) | CO₂-Selektivität (% bezogen auf die Menge umgesetzten Ethylens) |
| 1 | 746 | 8.8 |
| 2 | 742 | 8.9 |
| Vergleich | 715 | 9.5 |

## Patentansprüche

1. Verfahren zur Herstellung eines Katalysators zur Produktion von Vinylacetat in der Gasphase aus Ethylen, Essigsäure und Sauerstoff oder Sauerstoff enthaltenden Gasen, der Palladium und/oder dessen Verbindungen, Gold und/oder dessen Verbindungen sowie Alkalimetallverbindungen auf einem teilchenförmigen, porösen Träger enthält, wobei der Katalysator hergestellt wird, indem man
a) den Träger mit löslichen Palladium- und Goldverbindungen imprägniert,
b) die löslichen Palladium- und Goldverbindungen durch Zugabe einer alkalisch reagierenden Lösung auf dem Träger in unlösliche Palladium- und Goldverbindungen überführt,
c) die unlöslichen Palladium- und Goldverbindungen auf dem Träger durch ein Reduktionsmittel in flüssiger oder gasförmiger Phase reduziert,
d) den Träger mit mindestens einer löslichen Alkalimetallverbindung imprägniert und
e) den Träger abschließend bei höchstens 150°C trocknet,
**dadurch gekennzeichnet, daß** der Katalysator vor, während oder nach einem der Verfahrensschritte a) bis e) mit Mikrowellen bestrahlt wird.

2. Verfahren nach Anspruch 1, **dadurch gekennzeichnet, daß** die Bestrahlung des Katalysators bei einer Strahlungsfrequenz von 300 MHz bis 30 GHz, bevorzugt 2.45 GHz, und einer Strahlungsleistung von 10 bis 2000 W, bevorzugt 180-900 W, insbesondere 300-600 W, sowie eine Bestrahlungsdauer von bis zu 10 h, bevorzugt 1-60 min, insbesondere 5-15 min, erfolgt.

3. Verfahren nach Anspruch 1 oder 2, **dadurch gekennzeichnet, daß** der Katalysatorträger vor dem Imprägnierungschritt a) der Mikrowellenbestrahlung ausgesetzt wird.

4. Verfahren nach Anspruch 1 oder 2, **dadurch gekennzeichnet, daß** der Katalysator nach dem Imprägnierungsschritt a) und vor dem Fixierungsschritt b) mit den Mikrowellen bestrahlt wird.

5. Verfahren nach Anspruch 1 oder 2, **dadurch gekennzeichnet, daß** die Mikrowellenbestrahlung während des Reduktionschrittes c) erfolgt, vorzugsweise dann, wenn die Reduktion in der Gasphase durchgeführt wird.

6. Verfahren nach Anspruch 1 oder 2, **dadurch gekennzeichnet, daß** der Katalysator im Anschluß an Schritt e) mit Mikrowellen bestrahlt wird.

7. Verfahren nach Anspruch 1, **dadurch gekennzeichnet, daß** in Schritt c) die unlöslichen Palladium- und Gold-verbindungen auf dem Träger entweder in flüssiger Phase bei einer Temperatur von 0-90°C, vorzugsweise 15-25°C, mit Hydrazin, Ameisensäure oder einem Alkaliborhydrid, bevorzugt Natriumborhydrid, als Reduktionsmittel behandelt werden oder aber in der Gasphase bei einer Temperatur von 40 - 260 °C, vorzugsweise 70 - 200°C mit Wasserstoff, Ethylen, Propylen, Isobutylen, Butylen als Reduktionsmittel.

8. Verfahren nach Anspruch 1, **dadurch gekennzeichnet, daß** der Träger Schritt d) mit einer wäßrigen Lösung einer Alkalimetallverbindung, vorzugsweise einem Alkalimetallcarboxylat, insbesondere Kaliumacetat, imprägniert wird.

9. Katalysator zur Herstellung von Vinylacetat in der Gasphase aus Ethylen, Essigsäure und Sauerstoff oder Sauerstoff enthaltenden Gasen, der Palladium und/oder dessen Verbindungen, Gold und/oder dessen Verbindungen sowie Alkalimetallverbindungen auf einem teilchenförmigen, porösen Träger enthält, erhältlich, indem man
a) den Träger mit löslichen Palladium- und Goldverbindungen imprägniert,
b) die löslichen Palladium- und Goldverbindungen durch Zugabe einer alkalisch reagierenden Lösung auf dem Träger in unlösliche Palladium- und Goldverbindungen überführt,
c) die unlöslichen Palladium- und Goldverbindungen auf dem Träger durch ein Reduktionsmittel in flüssiger oder gasförmiger Phase reduziert,
d) den Träger mit mindestens einer löslichen Alkalimetallverbindung imprägniert und
e) den Träger abschließend bei höchstens 150°C trocknet,
**dadurch gekennzeichnet, dass** der Katalysator nach dem Verfahrensschritt e) mit Mikrowellen bestrahlt wird.

10. Katalysator nach Anspruch 9, **dadurch gekennzeichnet, daß** er, bezogen auf die Gesamtmasse des Katalysators, 0.2 - 2.5 Gew%, vorzugsweise 0.6 - 1.5 Gew%, Palladium, 0.2 - 2.5 Gew%, vorzugsweise 0.3 - 1.0 Gew%, Gold, 0.1 - 10 Gew% Alkalimetall, vorzugsweise 1.0 - 4.0 Gew% Alkalimetall, insbesondere Kalium, enthält.

11. Verfahren zur Herstellung von Vinylacetat in der Gasphase aus Ethylen, Essigsäure und Sauerstoff und/oder Sauerstoff enthaltenden Gasen in Gegenwart des Katalysators nach Anspruch 9 oder 10.

12. Verfahren nach Anspruch 11, **dadurch gekennzeichnet, daß** es bei Temperaturen von 100 bis 220 °C, vorzugsweise 120 bis 200 °C, und Drücken von 0,1 bis 2,5 MPa, vorzugsweise 0,1 bis 2 MPa, durchgeführt wird.

## Claims

1. A process for producing a catalyst for the preparation of vinyl acetate in the gas phase from ethylene, acetic acid and oxygen or oxygen-containing gases, which catalyst comprises palladium and/or its compounds, gold and/or its compounds and also alkali metal compounds on a particulate, porous support and is produced by
a) impregating the support with soluble palladium and gold compounds,
b) converting the soluble palladium and gold compounds into insoluble palladium and gold compounds by addition of an alkaline solution to the support,
c) reducing the insoluble palladium and gold compounds on the support by means of a reducing agent in the liquid or gaseous phase,
d) impregnating the support with at least one soluble alkali metal compound and
e) finally drying the support at a maximum of 150°C,
wherein the catalyst is irradiated with microwaves before, during or after one of the process steps a) to e).

2. The process as claimed in claim 1, wherein the irradiation of the catalyst is carried out at a radiation frequency of from 300 MHz to 30 GHz, preferably at 2.45 GHz, and a microwave power of from 10 to 2000 W, preferably 180-900 W, in particular 300-600 W and an irradiation time of up to 10 hours, preferably 1-60 min, in particular 5-15 min.

3. The process as claimed in claim 1 or 2, wherein the catalyst support is subjected to the microwave irradiation prior to the impregnation step a).

4. The process as claimed in claim 1 or 2, wherein the catalyst is irradiated with the microwaves after the impregnation step a) and before the fixing step b).

5. The process as claimed in claim 1 or 2, wherein microwave irradiation is carried out during the reduction step c), preferably when the reduction is carried out in the gas phase.

6. The process as claimed in claim 1 or 2, wherein the catalyst is irradiated with microwaves after step e).

7. The process as claimed in claim 1, wherein, in step c), the insoluble palladium and gold compounds on the support are either treated in the liquid phase at a temperature of 0-90°C, preferably 15-25°C, with hydrazine, formic acid or an alkali metal borohydride, preferably sodium borohydride, as reducing agent or else in the gas phase at a temperature of 40-260°C, preferably 70-200°C, with hydrogen, ethylene, propylene, isobutylene or butylene as reducing agent.

8. The process as claimed in claim 1, wherein, in step d), the support is impregnated with an aqueous solution of an alkali metal compound, preferably an alkali metal carboxylate, in particular potassium acetate.

9. A catalyst for preparing vinyl acetate in the gas phase from ethylene, acetic acid and oxygen or oxygen-containing gases, which comprises palladium and/or its compounds, gold and/or its compounds and also alkali metal compounds on a particulate, porous support, obtainable by
a) impregating the support with soluble palladium and gold compounds,
b) converting the soluble palladium and gold compounds into insoluble palladium and gold compounds by addition of an alkaline solution to the support,
c) reducing the insoluble palladium and gold compounds on the support by means of a reducing agent in the liquid or gaseous phase,
d) impregnating the support with at least one soluble alkali metal compound and
e) finally drying the support at a maximum of 150°C,
wherein the catalyst is irradiated with microwaves after the process step e).

10. A catalyst as claimed in claim 9 which comprises, based on the total mass of the catalyst, 0.2-2.5% by weight, preferably 0.6-1.5% by weight of palladium, 0.2-2.5% by weight, preferably 0.3-1.0% by weight, of gold and 0.1-10% by weight of alkali metal, preferably 1.0-4.0% by weight of alkali metal, in particular potassium.

11. A process for preparing vinyl acetate in the gas phase from ethylene, acetic acid and oxygen and/or oxygen-containing gases in the presence of the catalyst as claimed in claim 9 or 10.

12. The process as claimed in claim 11 carried out at temperatures of from 100 to 220°C, preferably from 120 to 200°C, and pressures of from 0.1 to 2.5 MPa, preferably from 0.1 to 2 MPa.

## Revendications

1. Procédé pour la préparation d'un catalyseur destiné à la production d'acétate de vinyle en phase gazeuse à partir d'éthylène, d'acide acétique et d'oxygène ou de gaz contenant de l'oxygène, qui contient du palladium et/ou ses composés, de l'or et/ou ses composés ainsi que des composés de métal alcalin sur un support poreux, en forme de particules, le catalyseur étant préparé en ce qu'on
a) imprègne le support avec les composés solubles de palladium et d'or,
b) transforme les composés solubles de palladium et d'or en composés insolubles de palladium et d'or sur le support par addition d'une solution à réaction alcaline,
c) réduit les composés de palladium et d'or insolubles sur le support par un réducteur en phase liquide ou gazeuse,
d) imprègne le support avec au moins un composé de métal alcalin soluble et
e) sèche ensuite le support à au maximum 150°C,
**caractérisé en ce que** le catalyseur est irradié avant, pendant ou après une des étapes de procédé a) à e) par des micro-ondes.

2. Procédé selon la revendication 1, **caractérisé en ce que** l'irradiation du catalyseur est réalisée à une fréquence d'irradiation de 300 MHz à 30 GHz, de préférence de 2,45 GHz, et une puissance d'irradiation de 10 à 2000 W, de préférence de 180 à 900 W, en particulier de 300 à 600 W, ainsi que pendant une durée d'irradiation allant jusqu'à 10 h, de préférence de 1 à 60 min, en particulier de 5 à 15 min.

3. Procédé selon la revendication 1 ou 2, **caractérisé en ce que** le support de catalyseur est soumis à une irradiation par des micro-ondes avant l'étape d'imprégnation a).

4. Procédé selon la revendication 1 ou 2, **caractérisé en ce que** le catalyseur est irradié par les micro-ondes après l'étape d'imprégnation a) et avant l'étape de fixation b).

5. Procédé selon la revendication 1 ou 2, **caractérisé en ce que** l'irradiation par les micro-ondes est réalisée pendant l'étape de réduction c), de préférence lorsque la réduction est réalisée en phase gazeuse.

6. Procédé selon la revendication 1 ou 2, **caractérisé en ce que** le catalyseur est irradié par les micro-ondes consécutivement à l'étape e).

7. Procédé selon la revendication 1, **caractérisé en ce que**, dans l'étape c), les composés insolubles de palladium et d'or sur le support sont traités en phase liquide à une température de 0 à 90°C, de préférence de 15 à 25°C, avec de l'hydrazine, de l'acide formique ou un borohydrure alcalin, de préférence le borohydrure de sodium, comme réducteur ou en phase gazeuse à une température de 40 à 260°C, de préférence de 70 à 200°C avec de l'hydrogène, de l'éthylène, du propylène, de l'isobutylène, du butylène comme réducteurs.

8. Procédé selon la revendication 1, **caractérisé en ce que** le support est imprégné dans l'étape d) avec une solution aqueuse d'un composé de métal alcalin, de préférence d'un carboxylate de métal alcalin, en particulier d'acétate de potassium.

9. Catalyseur pour la préparation d'acétate de vinyle en phase gazeuse à partir d'éthylène, d'acide acétique et d'oxygène ou de gaz contenant de l'oxygène, qui contient du palladium et/ou ses composés, de l'or et/ou ses composés ainsi que des composés de métal alcalin sur un support poreux, en forme de particules, pouvant être obtenu en ce qu'on
a) imprègne le support avec les composés solubles de palladium et d'or,
b) transforme les composés solubles de palladium et d'or en composés insolubles de palladium et d'or sur le support par addition d'une solution à réaction alcaline,
c) réduit les composés de palladium et d'or insolubles sur le support par un réducteur en phase liquide ou gazeuse,
d) imprègne le support avec au moins un composé de métal alcalin soluble et
e) sèche ensuite le support à au maximum 150°C,
**caractérisé en ce que** le catalyseur est irradié par des micro-ondes après l'étape de procédé e).

10. Catalyseur selon la revendication 9, **caractérisé en ce qu'**il contient par rapport à la masse totale du catalyseur 0,2 à 2,5% en poids, de préférence 0,6 à 1,5% en poids, de palladium, 0,2 à 2,5% en poids, de préférence 0,3 à 1,0% en poids, d'or ainsi que 0,1 à 10% en poids de métal alcalin, de préférence 1,0 à 4,0% en poids de métal alcalin, en particulier le potassium.

11. Procédé pour la préparation d'acétate de vinyle en phase gazeuse à partir d'éthylène, d'acide acétique et d'oxygène ou de gaz contenant de l'oxygène en présence du catalyseur selon la revendication 9 ou 10.

12. Procédé selon la revendication 11, **caractérisé en ce qu'**il est réalisé à des températures de 100 à 220°C, de préférence de 120 à 200°C, et à des pressions de 0,1 à 2,5 MPa, de préférence de 0,1 à 2 MPa.
